# EUROPÄISCHE PATENTANMELDUNG

(11) **EP 4 406 500 A1**
(43) Veröffentlichungstag der Anmeldung: **31.07.2024**
(21) Anmeldenummer: 23153318.3
(22) Anmeldetag: 25.01.2023
(51) Int. Cl.: A61B 18/12

(54) **CHIRURGIESYSTEM UND COMPUTER-IMPLEMENTIERTES VERFAHREN ZU DESSEN BETRIEB**

(71) Anmelder: Erbe Elektromedizin GmbH, 72072 Tübingen (DE)
(72) Erfinder: SELIG, Peter, 72147 Nehren (DE); DANIEL, Yannick, 72336 Balingen (DE); SCHLEEHAUF, Sebastian, 72074 Tuebingen (DE)
(74) Vertreter: Rüger Abel Patentanwälte PartGmbB

(57) **Zusammenfassung**

Ein Chirurgiesystem (10) und Verfahren (C) zu dessen Betrieb. Das Chirurgiesystem (10) beinhaltet ein Chirurgieinstrument (12), das an ein Chirurgiegerät (11) angeschlossen und von diesem gesteuert wird. Das Chirurgiegerät (11) steuert wenigstens einen Betriebsparameter (B) des Chirurgieinstruments (12). Der Betriebsparameter (B) kann ein Parameter eines elektrischen Ausgangssignals sein, wie beispielsweise eine Amplitude und/oder eine Frequenz und/oder eine Kurvenform. Das Chirurgiesystem (10) hat eine Auswerteeinheit (28), die dazu eingerichtet ist, eine aktuell durchgeführte oder geplante Verwendungsart (V) des Chirurgieinstruments (12) bzw. des gesamten Chirurgiesystems (10) zu identifizieren, wobei die Verwendungsart (V) charakteristisch ist für die Art der durchzuführenden Operation. Der identifizierten Verwendungsart (V) ist wenigstens ein Musterparameter (M) zugeordnet, der mit jeweils einem zugeordneten Betriebsparameter (B) verglichen werden kann. Basierend auf dem Resultat des Vergleichs kann wenigstens eine Maßnahme (X) eingeleitet werden, beispielsweise Ausgeben einer Information (INF) und/oder Anpassen wenigstens eines Betriebsparameters (B).

## Beschreibung

Die Erfindung betrifft ein Chirurgiesystem aufweisend ein Chirurgieinstrument und ein Chirurgiegerät, das zum Anschließen eines Chirurgieinstruments eingerichtet ist. Bei dem Chirurgieinstrument handelt es sich insbesondere um ein elektrochirurgisches Instrument mit wenigstens einer Elektrode. Alternativ oder zusätzlich kann das Chirurgieinstrument auch ein Ultraschallinstrument oder ein Wasserstrahl-Chirurgieinstrument sein. Das Chirurgieinstrument kann rein elektrisch betrieben werden und hierfür mit einer elektrischen Ausgangsleistung über das Chirurgiegerät versorgt werden. Zusätzlich oder alternativ kann wenigstens ein Fluid vom Chirurgiegerät zum Betrieb des Chirurgieinstruments bereitgestellt werden, beispielsweise ein Gas zur Erzeugung eines Plasmas, wie z.B. Argon oder ein anderes Inertgas und/oder eine Flüssigkeit, wie z.B. Wasser für eine Wasserstrahl-Chirurgie.

Im Stand der Technik sind unterschiedliche Ansätze verfolgt worden, um den Betrieb solcher Chirurgiesysteme zu verbessern oder zu optimieren.

US 2018/028088 A1 schlägt vor, verschiedene Überwachungsdaten aus einem Operationssaal zu verwenden, um den Ablauf einer Operation zu verfolgen und gegebenenfalls auf Operationsrisiken hinzuweisen. Hierfür werden im Operationssaal unterschiedliche Überwachungssensoren eingesetzt, z.B. Elektroenzephalographie-Sensoren (EEG-Sensoren), mit denen das Operationsteam ausgestattet ist oder Überwachungskameras im Operationssaal. Die Daten der Überwachungssensoren können mit statistischen Methoden ausgewertet werden.

Aus WO 2022/157695 A1 ist ein Überwachungsverfahren einer Operation bekannt. Bei dem Verfahren wird ein Chirurgiesystem eingesetzt, bei dem Betriebsdaten eines Instruments und Sensordaten einer das Instrument anwendenden Bedienperson an eine Auswerteeinheit übermittelt werden. Die Aktionen der Bedienperson werden bewertet und es wird eine Rückmeldung an die Bedienperson erzeugt, die daraufhin die Anwendung des Instruments anpassen kann. Beispielsweise können an der Bedienperson Bewegungs- oder Biomarker angeordnet sein, die von einem entsprechenden Erfassungssystem erkannt werden können.

EP 3 895 176 A1 offenbart ein Verfahren und ein System zum Extrahieren einer tatsächlichen Dauer eines chirurgischen Eingriffs. Dabei wird als tatsächlicher chirurgischer Eingriff die Summe der Zeitdauern verstanden, während denen ein Patient mit einem chirurgischen Werkzeug behandelt wird. Um diese Dauer zu ermitteln, wird die Gesamtdauer eines chirurgischen Verfahrens ermittelt und hiervon die Zeitdauern abgezogen, während denen keine chirurgische Behandlung stattfindet.

Zusätzlich zur Überwachung eines Operationsablaufs ist es auch bekannt, wenigstens einen Betriebsparameter eines Chirurgiesystems zu überwachen und basierend darauf eine Einstellung des Chirurgiesystems anzupassen. Einen solchen Ansatz beschreibt z.B. EP 3 876 238 A1. Dadurch sollen insbesondere durch das Chirurgiesystem bereitgestellte Modes zum Betreiben des angeschlossenen Chirurgieinstruments angepasst, verworfen oder neu kreiert werden.

Ein System zur Verbesserung der Effizienz elektrochirurgischer Generatoren geht aus EP 3 167 834 A1 hervor. Der Generator hat einen Wechselrichter zur Erzeugung eines HF-Signals für ein angeschlossenes elektrochirurgisches Instrument. Der Leistungswirkungsgrad des Wechselrichters wird ermittelt. Die Steuerung des Wechselrichters wird durch eine Anzahl von Ausschaltperioden ergänzt, wenn der Leistungswirkungsgrad des Wechselrichters einen Schwellenwert erreicht. Dadurch soll die Effizienz des Generators bei niedrigen Ausgangsleistungsniveaus verbessert werden.

In DE 10 2009 024 612 A1 ist ein Chirurgiesystem mit einem Chirurgiegerät zur Bereitstellung einer HF-Ausgangsspannung sowie ein daran angeschlossenes elektrochirurgisches Instrument beschrieben. Um die Betriebsparameter für das elektrochirurgische Instrument ausreichend genau einzustellen, wird die in Abhängigkeit vom behandelten Gewebe variierende elektrische Last ermittelt. Abhängig von dieser Last wird ein am Chirurgieinstrument haptisch wahrnehmbares Signal für die Bedienperson erzeugt. Die Bedienperson kann daraufhin die Anwendung des Chirurgieinstruments anpassen, wie z.B. die Schnittgeschwindigkeit und/oder die Schnitttiefe.

Ziel der vorliegenden Erfindung ist es, den Einsatz eines Chirurgiesystems weiter zu verbessern. Es kann als Aufgabe der vorliegenden Erfindung angesehen werden, die Verwendung des Chirurgiesystems bei einer aktuellen Anwendung und/oder für zukünftige Anwendungen zu optimieren.

Das Chirurgiesystem gemäß der vorliegenden Erfindung weist ein Chirurgiegerät und ein daran angeschlossenes Chirurgieinstrument auf. Das Chirurgieinstrument ist insbesondere ein Elektrochirurgieinstrument. Das Chirurgiegerät ist dazu eingerichtet, das angeschlossene Chirurgieinstrument mit der erforderlichen elektrischen Leistung und/oder den erforderlichen Betriebsmedien zu versorgen. Elektrische Leistung bzw. Energie kann als hochfrequentes (HF-) Ausgangssignal bereitgestellt werden. Abhängig von der Ausgestaltung des Chirurgieinstruments können zusätzlich oder alternativ zu elektrischer Energie Betriebsmedien, wie ein Gas oder eine Flüssigkeit, bereitgestellt werden. Beispielsweise kann das Gas zur Plasmaerzeugung am Chirurgieinstrument benötigt und verwendet werden. Zur Versorgung des Chirurgieinstruments mit elektrischer Leistung und/oder einem Betriebsmedium kann das Chirurgiegerät eine Versorgungseinheit aufweisen.

Das Chirurgiegerät hat außerdem eine Steuereinheit. Die Steuereinheit kann beispielsweise dazu eingerichtet sein, die Versorgungseinheit und/oder das Chirurgieinstrument unmittelbar zu steuern. Mittels der Steuereinheit kann wenigstens ein Betriebsparameter des Chirurgieinstruments eingestellt werden.

Das Chirurgiesystem hat außerdem eine Auswerteeinheit. Die Auswerteeinheit kann zumindest teilweise Bestandteil des Chirurgiegeräts sein. Die Auswerteeinheit kann auch teilweise oder vollständig außerhalb des Chirurgiegeräts realisiert sein. In jedem Fall ist die Auswerteeinheit mit dem Chirurgiegerät kommunikationsverbunden.

Die Auswerteeinheit ist dazu eingerichtet, eine aktuell durchgeführte Anwendungsart oder Verwendungsart des Chirurgieinstruments oder eine bevorstehende, geplante Anwendungsart oder Verwendungsart des Chirurgieinstruments zu identifizieren. Unter der Anwendungsart oder Verwendungsart des Chirurgieinstruments wird insbesondere die Art des chirurgischen Eingriffs verstanden, der mit dem Chirurgiesystem bzw. dem Chirurgieinstrument durchgeführt wird, also die Art der chirurgischen Behandlung. Die Anwendungsart, insbesondere die Art des chirurgischen Eingriffs oder der chirurgischen Behandlung, ist gekennzeichnet durch einen Gewebetyp oder durch mehrere Gewebetypen, die während des chirurgischen Eingriffs behandelt werden, z.B. geschnitten, koaguliert, fusioniert, usw. Dabei kann die Anwendungsart des Chirurgiesystems gekennzeichnet sein durch eine Reihenfolge oder einen zeitlichen Verlauf der Einwirkung auf unterschiedliche Gewebetypen. Die Anwendungsarten oder Verwendungsarten des Chirurgieinstruments können sich voneinander unterscheiden durch das chirurgische Fachgebiet (Gastroenterologie, Pneumologie, Gynäkologie, Urologie, Viszeralchirurgie, etc.), dem sie zugeordnet sind, und/oder durch den wenigstens einen behandelten Gewebetyp. Beispiele für Anwendungsarten oder Verwendungsarten des Chirurgieinstruments können sein: Biopsien oder Myotomien oder Ektomien jeweils an unterschiedlichen Gewebetypen (Haut und/oder Muskelgewebe und/oder Fettgewebe und/oder innere Organe, etc.), bronchiale Rekanalisation, usw.

Die Identifikation einer aktuell durchgeführten oder geplanten Verwendungsart kann auf der Erfassung wenigstens eines Betriebsparameters bei einer aktuell durchgeführten Verwendung des Chirurgieinstruments basieren. Beispielsweise können Anwendungsarten voneinander unterschieden werden, indem wenigstens ein Betriebsparameter ausgewertet wird. Beispielsweise kann eine Unterscheidung durch eine oder mehrere der folgenden Auswertungen ermöglicht werden:
- Auftreten verschiedener Einstellungen eines Betriebsparameters oder mehrerer Betriebsparameter;
- Reihenfolge des Auftretens verschiedener Einstellungen eines Betriebsparameters oder mehrerer Betriebsparameter;
- Zeitdauer einer oder mehrerer Einstellungen eines Betriebsparameters oder mehrerer Betriebsparameter;
- zeitlicher Verlauf eines Betriebsparameters oder mehrerer Betriebsparameter;
- Kombination von Einstellungen mehrerer Betriebsparameter zu einem jeweiligen Beobachtungszeitpunkt.

Zur Identifikation der Verwendungsart des Chirurgiesystems kann der wenigstens eine Betriebsparameter ermittelt und mit jeweils einem zugehörigen Musterparameter eines oder mehrerer Verwendungsmuster verglichen werden. Wird eine ausreichende Ähnlichkeit zwischen dem wenigstens einen Betriebsparameter der aktuellen Anwendung und dem wenigstens einen Musterparameter eines Verwendungsmusters festgestellt, kann daraus geschlossen werden, dass die aktuelle Verwendungsart des Chirurgieinstruments mit der Verwendungsart übereinstimmt, die zu dem Verwendungsmuster gehört. Mit anderen Worten entspricht die aktuelle Verwendungsart der bekannten Verwendungsart des ausreichend ähnlichen Verwendungsmusters. Auf diese Weise ist es möglich, die Verwendungsart der aktuell durchgeführten Anwendung des Chirurgieinstruments zu identifizieren. Bei dieser Art der Identifikation ist es nicht erforderlich, dass dem Chirurgiesystem die Verwendungsart separat übermittelt wird, beispielsweise durch eine Eingabe über eine Bedienschnittstelle des Chirurgiesystems oder durch Übermittlung der Anwendungsart durch ein Datenmanagementsystem, an das das Chirurgiesystem angeschlossen sein kann.

Zur Prüfung der Ähnlichkeit können bekannte Methoden verwendet werden, wie sie z.B. auch auf dem Gebiet der Mustererkennung eingesetzt werden. Eine ausreichende Ähnlichkeit wird festgestellt, wenn der wenigstens eine Betriebsparameter identisch ist mit dem jeweils zugeordneten Musterparameter und/oder die Unterschiede innerhalb eines Toleranzbereichs liegen.

Zusätzlich oder alternativ ist es auch möglich, die geplante Anwendungsart für einen bevorstehenden chirurgischen Eingriff vorzugeben. Hierzu kann die Bedienperson, z.B. über eine Bedienschnittstelle des Chirurgiesystems, insbesondere eine Bedienschnittstelle des Chirurgiegeräts, eine geplante Verwendungsart eingeben, insbesondere die Art der durchzuführenden chirurgischen Behandlung. Die Verwendungsart kann auch in einem Datenmanagementsystem (z.B. Datenbank- und/oder Datenverarbeitungssystem), insbesondere einem Krankenhausinformationssystem (KIS) hinterlegt sein. Das Chirurgiesystem kann mittels eines Kommunikationsnetzwerks mit dem Datenmanagementsystem kommunikationsverbunden sein und die geplante Verwendungsart aus dem Datenmanagementsystem abrufen oder vom Datenmanagementsystem automatisch übermittelt bekommen. Das Datenmanagementsystem kann drahtlos und/oder drahtgebunden mit dem Chirurgiesystem und insbesondere mit dem Chirurgiegerät kommunikationsverbunden sein. Das Chirurgiegerät kann hierzu eine entsprechende Kommunikationsschnittstelle aufweisen.

Wenn die Auswerteeinheit des Chirurgiegeräts die Identifikation der Verwendungsart basierend auf einer aktuellen Anwendung des Chirurgieinstruments ermittelt, erfolgt die Identifikation zumindest etwas zeitverzögert nach dem Beginn der Verwendung des Chirurgieinstruments. In der Regel kann die Identifikation der Verwendungsart basierend auf dem wenigstens einen Betriebsparameter und dem Mustervergleich mit dem wenigstens einen Verwendungsmuster ausreichend früh im Laufe eines chirurgischen Eingriffs erfolgen, so dass die Verwendungsart zumindest für einen noch durchzuführenden Teil und insbesondere einen überwiegenden Teil des gesamten chirurgischen Eingriffs bekannt ist.

Nachdem die Verwendungsart identifiziert wurde, kann der wenigstens eine Betriebsparameter des Chirurgieinstruments mit dem wenigstens einen Musterparameter eines Verwendungsmusters verglichen werden. Das Verwendungsmuster ist derselben Verwendungsart zugeordnet wie die geplante oder aktuelle Anwendung des Chirurgiesystems. Das Verwendungsmuster kann beispielsweise eine übliche oder ideale Verwendung für die identifizierte Verwendungsart sein. Abhängig von einem Resultat des Vergleichs kann eine durchzuführende Maßnahme von der Auswerteeinheit ausgelöst bzw. durchgeführt werden. Beispielsweise kann eine Abweichung zwischen einem oder mehreren Betriebsparametern vom jeweils zugeordneten Musterparameter angezeigt werden, beispielsweise quantitativ und/oder qualitativ. Basierend darauf kann optional auch eine Bewertung der aktuellen Anwendung erfolgen.

Die wenigstens eine Maßnahme, die vom Chirurgiegerät und insbesondere der Auswerteeinheit ausgelöst oder durchgeführt werden kann, kann beispielsweise das Ausgeben einer Information an eine Bedienperson oder ein Mitglied des Operationsteams sein. Die Information kann als optische Information auf einem Bildschirm und/oder als akustische Information über einen Lautsprecher und/oder als haptische Information am Chirurgieinstrument ausgegeben werden. Die Information kann eine Information oder mehrere der nachfolgenden Informationen in beliebiger Kombination enthalten:
- eine Warnung, wenn eine Abweichung des wenigstens einen Betriebsparameters von dem jeweils zugeordneten Musterparameters vorliegt;
- eine Empfehlung für eine geänderte Einstellung des Betriebsparameters oder von wenigstens einem der Betriebsparameter;
- eine Information betreffend einen oder mehrere bevorstehende Schritte, die im Rahmen des chirurgischen Eingriffs erforderlich sind;

Die wenigstens eine Maßnahme kann zusätzlich oder alternativ umfassen, einen der Betriebsparameter oder mehrere der Betriebsparameter automatisch zu modifizieren oder einen Vorschlag für eine Anpassung eines oder mehrerer der Betriebsparameter anzuzeigen.

Basierend auf dem Resultat des Vergleichs kann der Bedienperson und/oder einem Mitglied des Chirurgieteams und/oder einer Planungsinstanz als wenigstens eine Maßnahme eine Information ausgegeben werden. Eine solche Information kann z.B. eine oder mehrere der folgenden Aspekte umfassen:
- eine ermittelte verbleibende Zeitdauer bis zum Abschluss der Anwendung bzw. des chirurgischen Eingriffs;
- eine ermittelte Zeitdauer für wenigstens einen folgenden Anwendungsschritt der Anwendung bzw. des chirurgischen Eingriffs;
- eine für wenigstens einen folgenden Anwendungsschritt benötigte Ressource (Material und/oder Person).
Diese Information kann sowohl ermittelt und ausgegeben werden, wenn bei dem Vergleich eine Abweichung zwischen wenigstens einem Betriebsparameter und wenigstens einem Musterparameter ergeben hat oder auch dann, wenn eine derartige Abweichung nicht ermittelt wurde. Basierend auf dieser Information kann z.B. die Belegung eines Operationssaals, Reinigungs- und Sterilisationsabläufe, Medikamenten- oder Instrumentenbedarf, Personaleinsatz oder irgendeiner anderen Ressource geplant und/oder angepasst werden.

Das Ermitteln und Initiieren bzw. Durchführen wenigstens einer geeigneten Maßnahme basiert gemäß der vorliegenden Erfindung auf der Identifikation der aktuellen oder geplanten Verwendungsart, also insbesondere auf der aktuell durchgeführten oder geplanten Art der Behandlung unter Verwendung des Chirurgiesystems. Die wenigstens eine Maßnahme kann daher sehr genau an die aktuelle Situation und insbesondere die aktuelle chirurgische Behandlung angepasst sein, was wiederum einen deutlichen Mehrwert des Chirurgiesystems darstellt. Beispielsweise kann die Gefahr von falschen Einstellungen von Betriebsparametern reduziert oder vollständig eliminiert werden. Unerwünschte Beeinträchtigungen des Gewebes während einer Behandlung lassen sich reduzieren oder vermeiden.

Die wenigstens eine Maßnahme kann auch das automatische Anpassen des Betriebsparameters oder von einem der Betriebsparameter umfassen.

Der wenigstens eine Betriebsparameter kann ein elektrische Ausgangssignal und/oder ein optisches Ausgangssignal und/oder ein Fluidparameter und/oder ein Leistungsparameter sein, das dem Chirurgieinstrument vom Chirurgiegerät zur Verfügung gestellt wird. Als Betriebsparameter können beispielsweise ein oder mehrere der nachfolgenden Parameter verwendet werden:
- eine Amplitude und/oder eine Frequenz eines elektrischen Ausgangssignals, insbesondere ein Hochfrequenzausgangssignals, beispielsweise einer Ausgangsspannung und/oder eines Ausgangsstroms;
- ein Crest-Faktor des elektrischen Ausgangssignals;
- ein Tastgrad des elektrischen Ausgangssignals, wenn es sich beim elektrischen Ausgangssignal um ein pulsweitenmoduliertes elektrisches Ausgangssignal handelt;
- eine Kurvenform des elektrischen Ausgangssignals oder eines Abschnitts (z.B. einer Halbwelle) des elektrischen Ausgangssignals;
- ein Mode des elektrischen Ausgangssignals;
- ein Fluidparameter eines dem Chirurgieinstrument bereitgestellten Fluids, beispielsweise ein Fluiddruck und/oder ein Volumenstrom;
- ein optisches Ausgangssignal.

Bei dem elektrischen Ausgangssignal kann es sich um eine elektrische Ausgangsspannung und/oder einen elektrischen Ausgangsstrom handeln. Der Betriebsparameter kann optional nur für die Ausgangsspannung oder nur für den Ausgangsstrom oder sowohl für die Ausgangsspannung als auch für den Ausgangsstrom eingestellt werden.

Bei einem Ausführungsbeispiel ist die Auswerteeinheit außerdem dazu eingerichtet, als das Resultat oder als eines der Resultate des Vergleichs eine Abweichung zwischen dem Betriebsparameter oder wenigstens einem der verwendeten Betriebsparameter bei der aktuellen Verwendung des Chirurgieinstruments gegenüber dem jeweils zugeordneten Musterparameter zu ermitteln. Die wenigstens eine Maßnahme wird insbesondere abhängig von der Abweichung ermittelt und initiiert. Es ist insbesondere vorteilhaft, wenn die Maßnahme oder eine der Maßnahmen nur dann ermittelt und initiiert wird, wenn die Abweichung ein Abweichungskriterium erfüllt. Das Abweichungskriterium kann ein quantitatives oder qualitatives Abweichungskriterium sein. Beispielsweise ist es möglich, eine bestimmte Maßnahme nur dann zu initiieren, wenn die ermittelte Abweichung größer ist als ein vom Abweichungskriterium vorgegebener Schwellenwert.

Während der Verwendung des Chirurgieinstruments kann beispielsweise der Betriebsparameter oder einer der einstellbaren Betriebsparameter kontinuierlich oder zeitdiskret zu vorgegebenen Überwachungszeitpunkten erfasst und gespeichert werden. Auf diese Weise kann eine zeitliche Reihenfolge der Einstellung von einem oder mehreren Betriebsparametern und/oder ein kontinuierlicher zeitlicher Verlauf des Betriebsparameters ermittelt und optional gespeichert werden.

Bevorzugt ist das Chirurgiegerät dazu eingerichtet, den Betriebsparameter oder wenigstens einen der Betriebsparameter bei der Verwendung des Chirurgieinstruments zu speichern und/oder an das Datenmanagementsystem zu übertragen, sofern das Chirurgiesystem mit dem Datenmanagementsystem kommunikationsverbunden ist. Die Kommunikationsverbindung kann auch temporär hergestellt werden, so dass zwischengespeicherte Betriebsparameter während der hergestellten Kommunikationsverbindung übermittelt werden können. Zusätzlich oder alternativ kann das Chirurgiesystem und insbesondere das Chirurgiegerät eine Schnittstelle (z.B. USB-Schnittstelle) aufweisen, an die ein Speichermedium angeschlossen werden kann, um gespeicherte Daten des wenigstens einen Betriebsparameters auf ein externes Speichermedium zu übertragen.

Vorteilhafte Ausgestaltungen der Erfindung ergeben sich aus den abhängigen Ansprüchen, der Beschreibung und der Zeichnung. Nachfolgend werden bevorzugte Ausführungsbeispiele der Erfindung basierend auf der Zeichnung im Einzelnen erläutert. In der Zeichnung zeigen:
Figur 1 eine schematische Darstellung eines Ausführungsbeispiels eines Chirurgiesystems,
Figur 2 ein Flussdiagramm eines Ausführungsbeispiels eines Verfahrens zum Betreiben eines Chirurgiesystems, insbesondere des Chirurgiesystems aus Figur 1,
Figur 3 eine beispielhafte schematische Darstellung mehrere Musterparameter einer Musterverwendung eines elektrochirurgischen Instruments eines Chirurgiesystems und
Figur 4 eine beispielhafte schematische Darstellung wenigstens eines Betriebsparameters bei der Verwendung eines elektrochirurgischen Instruments abhängig von dem Zeitpunkt.

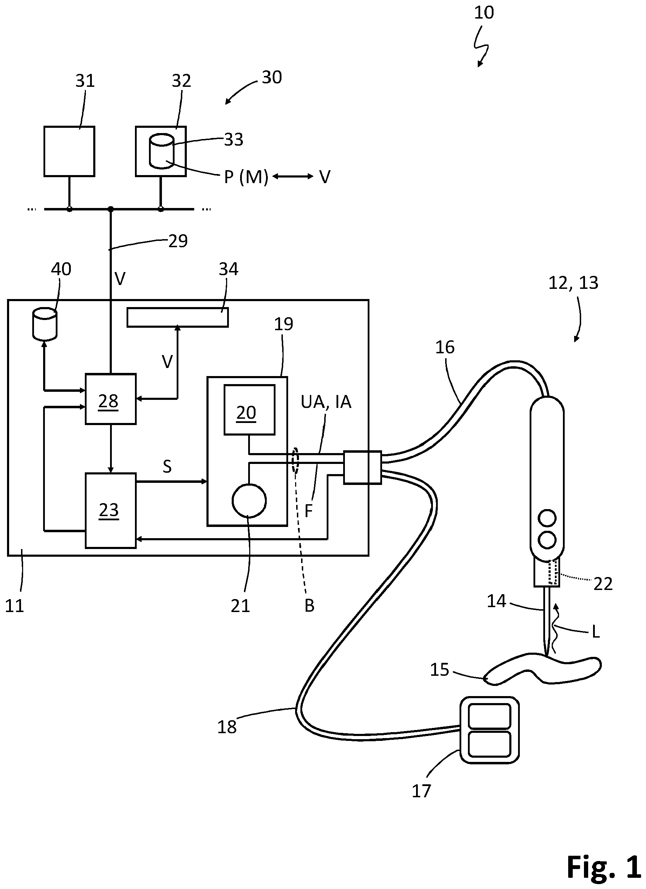

In Figur 1 ist schematisch nach Art eines Blockschaltbildes ein Chirurgiesystem 10 veranschaulicht, aufweisend ein Chirurgiegerät 11 sowie ein an das Chirurgiegerät 11 angeschlossenes Chirurgieinstrument 12. Beim Ausführungsbeispiel ist das Chirurgieinstrument 12 ein Elektrochirurgieinstrument 13. Das Elektrochirurgieinstrument 13 hat wenigstens eine Elektrode 14. Mittels der wenigstens einen Elektrode 14 kann das Elektrochirurgieinstrument 13 auf ein Gewebe 15 eines Patienten einwirken.

Das Chirurgieinstrument 12 ist über eine Instrumentenleitung 16 mit dem Chirurgiegerät 11 verbunden. Die Instrumentenleitung 16 kann eine elektrische und/oder fluidische und/oder optische Verbindung zwischen dem Chirurgieinstrument 12 und dem Chirurgiegerät 11 bereitstellen. Im Falle eines Elektrochirurgieinstruments 13 wird zumindest eine elektrische Verbindung hergestellt.

Bei dem in Figur 1 dargestellten Ausführungsbeispiel ist das Elektrochirurgieinstrument 13 als monopolares Instrument ausgebildet. Wenn aufgrund der Behandlung mit dem Elektrochirurgieinstrument 13 ein Strom in das Gewebe 15 fließt, sorgt eine am Patienten angebrachte Neutralelektrode 17 dafür, dass ein geschlossener Behandlungsstromkreis hergestellt wird. Die Neutralelektrode 17 ist elektrisch leitend mit dem Patienten verbunden und außerdem über eine Neutralelektrodenleitung 18 mit dem Chirurgiegerät 11 elektrisch verbunden. Bei der Verwendung eines bipolaren Elektrochirurgieinstruments 13 kann die Neutralelektrode 17 entfallen.

Das Chirurgiegerät 11 ist dazu eingerichtet, das daran angeschlossene Chirurgieinstrument 12 mit elektrischer Leistung bzw. Energie und/oder wenigstens einem Betriebsmedium zu versorgen. Zur Versorgung weist das Chirurgiegerät 11 eine Versorgungseinheit 19 auf. Über die Versorgungseinheit 19 wird dem Chirurgieinstrument 12 ein elektrisches Ausgangssignal und/oder ein Fluid und/oder ein optisches Signal und/oder Energie in Form von Laserlicht, etc. bereitgestellt. Bei dem in Figur 1 veranschaulichten Ausführungsbeispiel weist die Versorgungseinheit 19 einen Generator 20 auf, um an die wenigstens eine Elektrode 14 des Elektrochirurgieinstruments 13 ein elektrisches Ausgangssignal anzulegen, das in Form einer elektrischen Ausgangsspannung UA und/oder eines elektrischen Ausgangsstromes IA bereitgestellt werden kann. Bei der elektrischen Ausgangsspannung UA und/oder dem elektrischen Ausgangsstrom IA handelt es sich insbesondere um ein hochfrequentes elektrisches Ausgangssignal in einem Frequenzbereich von beispielsweise 100 kHz oder 300 kHz bis 4 MHz. Optional kann die Versorgungseinheit 19 zusätzlich zum Generator 20 auch eine Gasquelle 21 aufweisen, um dem Elektrochirurgieinstrument 13 ein Gas zur Plasmabildung und/oder ein Spülgas bereitzustellen.

Das Chirurgieinstrument 12 kann außerdem wenigstens einen Sensor 22 aufweisen, um während der Anwendung des Chirurgieinstruments 12 ein Messsignal zu erzeugen, das einen zu messenden Parameter beschreibt, und über die Instrumentenleitung 16 an das Chirurgiegerät 11 und insbesondere eine Steuereinheit 23 des Chirurgiegeräts 11 zu übermitteln. Beispielsweise kann mittels des Sensors 22 Licht L erfasst werden, das bei der Einwirkung auf das Gewebe 15 mittels der wenigstens einen Elektrode 14 entsteht. Das Licht L kann beispielsweise durch eine Funkenbildung zwischen der wenigstens einen Elektrode 14 und dem Gewebe 15 emittiert werden. Das Messsignal kann in der Steuereinheit 23 analysiert werden. Beispielsweise kann eine Spektralanalyse des Lichts L durchgeführt werden. Aus dem Messsignal kann wenigstens ein Betriebsparameter B ermittelt werden. Basierend auf dem Messsignal und/oder einem daraus abgeleiteten Betriebsparameter kann der Betrieb des Chirurgieinstruments 12 somit überwacht werden und gegebenenfalls kann der Betriebsparameter B oder einer der Betriebsparameter B in einer geschlossenen Regelschleife geregelt werden.

Allgemein ist die Steuereinheit 23 zur Steuerung des wenigstens einen Betriebsparameters B eingerichtet, mit dem das Chirurgieinstrument 12 betrieben wird. Dieser Betriebsparameter B kann vom Chirurgiegerät 11 eingestellt werden. Hierzu kann die Steuereinheit 23 beispielsweise ein Steuersignal S an die Versorgungseinheit 19 übermitteln, um einen oder mehrere Betriebsparameter B vorzugeben bzw. einzustellen.

Das Chirurgiegerät 11 hat außerdem eine Auswerteeinheit 28. Die Auswerteeinheit 28 ist beispielsgemäß Bestandteil des Chirurgiegeräts 11, kann alternativ zu Figur 1 jedoch auch außerhalb des Chirurgiegeräts 11 angeordnet und mit dem Chirurgiegerät 11 kommunikationsverbunden sein, beispielsweise über ein Kommunikationsnetzwerk 29. Die Verbindung über das Kommunikationsnetzwerk 29 kann drahtgebunden oder drahtlos sein.

Die Auswerteeinheit 28 und die Steuereinheit 23 sind beispielsgemäß kommunikationsverbunden oder können alternativ zu der Darstellung in Figur 1 auch als gemeinsame Einheit oder Baugruppe realisiert sein. Der Auswerteeinheit 28 können auf diese Weise der durch die Steuereinheit 23 eingestellte und/oder vorgegebene Betriebsparameter B übermittelt werden. Die Auswerteeinheit 28 kann außerdem mittelbar über die Steuereinheit 23 oder alternativ auch unmittelbar mit dem Sensor 22 verbunden sein, so dass auch das vom Sensor 22 bereitgestellte Messsignal für die Auswerteeinheit 28 zur Verfügung stehen kann.

Die Auswerteeinheit 28 ist bei dem in Figur 1 dargestellten Ausführungsbeispiel des Chirurgiesystems 10 über das Kommunikationsnetzwerk 29 mit einem Datenmanagementsystem 30 kommunikationsverbunden. Das Datenmanagementsystem 30 kann eine oder mehrere externe Recheneinheiten 31 und/oder Datenbanken 32 aufweisen. Beim Ausführungsbeispiel enthält das Datenmanagementsystem 30 eine Datenbank 32, einen Datenbankspeicher 33. In dem Datenbankspeicher 33 sind mehrere Verwendungsmuster P abgespeichert. Jedem Verwendungsmuster P ist wenigstens ein Musterparameter M zugeordnet. Zumindest einer der Musterparameter M, mehrere der Musterparameter M oder alle Musterparameter M entsprechen jeweils einem zugeordneten Betriebsparameter B. Jedes Verwendungsmuster P ist durch den Wert der zugehörigen Musterparameter M definiert, wobei der Wert jedes Musterparameters M konstant oder zeitabhängig variierend sein kann. Jeder Musterparameter M kann beispielsweise eine zeitliche Abfolge verschiedener Werte in mittelbar oder unmittelbar aufeinanderfolgenden Zeitabschnitten sein.

Das Datenmanagementsystem 30 kann beispielsweise ein Krankenhausinformationssystem (KIS) sein. Zusätzlich zu dem Datenbankspeicher 33, der die Verwendungsmuster P enthält, können auch weitere Datenbankspeicher vorhanden sein, in denen beispielsweise Operationspläne oder sonstige Ressourcenplanungen, Patientendaten, usw. abgespeichert sein können. Das Chirurgiesystem 10 kann über das Kommunikationsnetzwerk 29 optional auf solche zusätzlichen Daten und Informationen zugreifen, die über das Datenmanagementsystem 30 (z.B. Krankenhausinformationssystem) bereitgestellt werden.

In einer abgewandelten Ausführungsform kann das Datenmanagementsystem 30 und/oder die wenigstens eine externe Recheneinheit 31 und/oder die wenigstens eine Datenbank 32 auch in das Chirurgiegerät 11 integriert sein bzw. innerhalb des Chirurgiegeräts 11 angeordnet sein (nicht in Fig. 1 dargestellt).

Als Betriebsparameter B können sämtliche Einstellungen verwendet werden, die den Betrieb des Chirurgieinstruments 12 charakterisieren, insbesondere solche Parameter, die durch das Chirurgiegerät 11 vorgegeben werden und optional vor und/oder während einer Anwendung veränderbar bzw. einstellbar sind. Als der wenigstens eine Betriebsparameter B können bei dem hier veranschaulichten Ausführungsbeispiel ein oder mehrere der folgenden Betriebsparameter B verwendet werden:
- die Amplitude der Ausgangsspannung UA;
- die Frequenz der Ausgangsspannung UA;
- die Kurvenform der Ausgangsspannung UA;
- ein Crest-Faktor (Scheitelfaktor) der Ausgangsspannung UA;
- die Amplitude des Ausgangsstromes IA;
- die Frequenz des Ausgangsstromes IA;
- die Kurvenform des Ausgangsstromes IA;
- ein Crest-Faktor (Scheitelfaktor) des Ausgangsstromes IA;
- eine Gewebeimpedanz des Gewebes 15;
- eine Impedanz eines Behandlungsstromkreises vom Chirurgiegerät 11 zum Chirurgieinstrument 12 und über das Gewebe 15 (und optional eine Neutralelektrode 17) zurück zum Chirurgiegerät 11;
- einen Fluiddruck eines von der Fluidquelle 21 bereitgestellten Fluids,
- einen Volumenstrom des von der Fluidquelle 21 bereitgestellten Fluids.

Das Chirurgiegerät 11 hat beim Ausführungsbeispiel eine Bedienschnittstelle 34. Über die Bedienschnittstelle kann eine Bedienperson Eingaben vornehmen und beispielsweise den wenigstens einen Betriebsparameter B einstellen. Hierfür ist die Bedienschnittstelle 34 mit der Steuereinheit 23 kommunikationsverbunden. Die Steuereinheit 23 kann die Bedienperson über die Bedienschnittstelle 34 über aktuelle Betriebszustände informieren und/oder andere Informationen ausgeben. Die Bedienschnittstelle 34 kann beispielsweise einen berührungsempfindlichen Bildschirm zur Eingabe und Ausgabe von Daten und Informationen aufweisen. Zusätzlich oder alternativ können auch andere Einrichtungen, wie beispielsweise ein Lautsprecher, Mikrofon, Tasten, Knöpfe, etc. Bestandteil der Bedienschnittstelle 34 sein. Die Auswerteeinheit 28 ist beim Ausführungsbeispiel unmittelbar oder mittelbar über die Steuereinheit 23 mit der Bedienschnittstelle 34 kommunikationsverbunden.

Dem Chirurgiesystem 10 kann über die Bedienschnittstelle 34 oder das Datenmanagementsystem 30 eine Information übermittelt werden, die eine geplante Verwendungsart V des Chirurgieinstruments 12 beschreibt. Die Verwendungsart V charakterisiert die Verwendung des Chirurgieinstruments 12 während der Behandlung bzw. während der Einwirkung auf Gewebe 15 eines Patienten. Die Verwendungsart V des Chirurgieinstruments 12 ist insbesondere gekennzeichnet durch wenigstens einen der nachfolgend genannten Parameter:
- eine Gesamtdauer DE des Chirurgieinstruments von einem Startzeitpunkt ts bis zu einem Endzeitpunkt te eines chirurgischen Eingriffs;
- eine Kombination oder Folge verschiedener Einstellungen eines Betriebsparameters B oder einer Gruppe mehrerer oder aller Betriebsparameter B;
- ein kontinuierlicher zeitlicher Verlauf eines Betriebsparameters B oder einer Gruppe mehrerer oder aller Betriebsparameter B;
- wenigstens einer Aktivierungsdauer DA während der Gesamtdauer DE, in denen das Chirurgieinstrument 12 aktiviert ist und auf das Gewebe 15 des Patienten einwirkt;
- wenigstens einer Deaktivierungsdauer DD während der Gesamtdauer DE, während der das Chirurgieinstrument 12 deaktiviert ist und nicht zur Beeinflussung des Gewebes 15 des Patienten verwendet wird, wobei eine Deaktivierungsdauer DD zwei aufeinanderfolgende Aktivierungsdauern DA voneinander trennen kann;
- ein von einem Betriebsparameter B abgeleiteter Parameter, beispielsweise eine Impedanz des Gewebes 15, die beispielsweise basierend auf der Ausgangsspannung UA und dem Ausgangsstrom IA ermittelt werden kann.

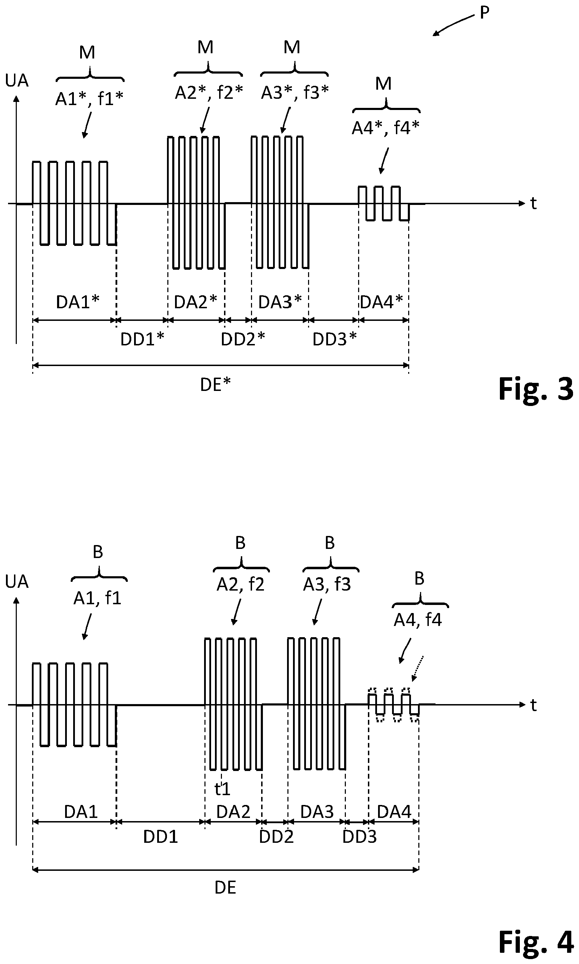

In Figur 4 ist beispielhaft ein zeitlicher Verlauf für die Ausgangsspannung UA während einer chirurgischen Behandlung veranschaulicht. Diese Darstellung ist lediglich schematisch beispielhaft zur Erläuterung des Erfindungsprinzips und kann abhängig von der Verwendungsart V des Chirurgieinstruments 12 variieren.

Der chirurgische Eingriff beginnt mit dem Startzeitpunkt ts und endet mit dem Endzeitpunkt te. Während der Gesamtdauer DE sind beispielhaft vier Aktivierungsdauern DA, nämlich eine erste Aktivierungsdauer DA1, eine zweite Aktivierungsdauer DA2, eine dritte Aktivierungsdauer DA3 und eine vierte Aktivierungsdauer DA4 vorgesehen. Zwischen zwei aufeinanderfolgenden Aktivierungsdauern ist jeweils eine Deaktivierungsdauer DD vorhanden, beim Ausführungsbeispiel eine erste Deaktivierungsdauer DD1, eine zweite Deaktivierungsdauer DD2 sowie eine dritte Deaktivierungsdauer DD3, während denen das Chirurgieinstrument 12 (im Ausführungsbeispiel Elektrochirurgieinstrument 13) nicht zur Beeinflussung von Gewebe 15 verwendet wird.

In jeder Aktivierungsdauer DA sind ein Betriebsparameter B oder ein Satz von mehreren Betriebsparametern B des Chirurgieinstruments 12 eingestellt. Lediglich beispielhaft sind hier als Betriebsparameter die Amplitude A der Ausgangsspannung UA sowie die Frequenz f der Ausgangsspannung UA dargestellt. Beim Ausführungsbeispiel ist die Einstellung der Ausgangsspannung UA wie folgt: eine erste Amplitude A1 und eine erste Frequenz f1 während der ersten Aktivierungsdauer DA1, eine zweite Amplitude A2 und eine zweite Frequenz f2 während der zweiten Aktivierungsdauer DA2, eine dritte Amplitude A3 und eine dritte Frequenz f3 während der dritten Aktivierungsdauer DA3 und eine vierte Amplitude A4 und eine vierte Frequenz f4 während der vierten Aktivierungsdauer DA4. Im Beispiel sind die zweite Amplitude A2 und die dritte Amplitude A3 gleich groß und außerdem können die zweite Frequenz f2 und die dritte Frequenz f3 gleichgroß sein. Die Amplituden und Frequenzen während der Aktivierungsdauern DA1- DA4 können jeweils gleichgroß oder verschieden groß sein, was von der Verwendungsart V abhängt.

Zusätzlich oder alternativ zu der Ausgangsspannung UA können auch ein oder mehrere andere Betriebsparameter B während der unterschiedlichen Aktivierungsdauern DA1-DA4 konstant oder zeitlich variierend eingestellt sein. Beispielsweise kann zusätzlich die Kurvenform der Ausgangsspannung UA in einer oder mehreren Aktivierungsdauern von dem beispielhaft dargestellten Rechteckverlauf abweichen.

Die zeitliche Abfolge bzw. der zeitliche Verlauf der Betriebsparameter B, mit denen das Chirurgieinstrument 12 während des chirurgischen Eingriffs verwendet wird, kennzeichnet eine Verwendungsart Vi (i=1, 2, 3, ..., n) aus mehreren möglichen Verwendungsarten V1, V2, V3, ... ,Vn. Beispielsweise kann unterschiedlichen Behandlungen (chirurgischer Eingriff an einem der inneren Organe oder an Hautgewebe oder an Muskelgewebe) jeweils eine Verwendungsart zugeordnet werden. Für verschiedene chirurgische Eingriffe bzw. Behandlungen eines Patienten stehen also unterschiedliche Verwendungsarten V zur Verfügung, die insbesondere durch die Einstellung der Betriebsparameter B bzw. deren jeweiligen zeitlichen Verlauf gekennzeichnet sind.

Wenn zum Startzeitpunkt ts der Verwendung des Chirurgieinstruments 12 nicht bekannt ist, welche Verwendungsart V aktuell stattfindet, kann die Verwendungsart V auch aus den Einstellungen und insbesondere dem zeitlichen Verlauf von einem Betriebsparameter B bzw. mehreren Betriebsparametern B erkannt werden. Hierzu können z.B. die zeitlichen Verläufe eines oder mehrerer Betriebsparameter B der aktuell durchgeführten Verwendungsart V (Figur 4) mit entsprechenden Musterparametern M eines oder mehrerer Verwendungsmuster P verglichen werden. Ist eine ausreichende Ähnlichkeit mit einem Verwendungsmuster P vorhanden, kann festgestellt werden, dass die aktuelle Verwendung eine Verwendungsart V ist, die durch das identische oder ausreichend ähnliche Verwendungsmuster P beschrieben ist. Hierzu können bekannte Verfahren (z.B. Mustererkennung) eingesetzt werden. Sobald der zeitliche Verlauf des wenigstens einen Betriebsparameters B der aktuell durchgeführten Verwendung des Chirurgieinstruments 12 eine Unterscheidung zwischen den verfügbaren Verwendungsmustern P ermöglicht, kann festgestellt werden, dass die Verwendungsart des übereinstimmenden Verwendungsmusters P der aktuellen Verwendungsart entspricht. Dies kann beispielsweise zu einem ersten Zeitpunkt t1 (Figur 4) der Fall sein, zudem eine ausreichende Ähnlichkeit mit dem Verwendungsmuster P aus Figur 3 festgestellt wurde und eine ausreichende Unterscheidbarkeit gegenüber anderen Verwendungsmustern vorliegt.

Bei der Prüfung der Ähnlichkeit zwischen den Betriebsparametern B einer aktuellen Verwendung und dem jeweils zugeordneten Musterparameter M können einzelne Parameter stärker gewichtet werden als andere. Beispielsweise können die Aktivierungsdauer DA1-DA4 und/oder sich während einer der Aktivierungsdauern DA1-DA4 ändernde Betriebsparameter B und/oder sich zumindest zwischen zwei der Aktivierungsdauern DA1-DA4 unterscheidende Betriebsparameter B bei der Ähnlichkeitsprüfung stärker berücksichtigt werden als Deaktivierungsdauern DD1-DD3. Für das Beispiel der in Figur 4 dargestellten Verwendung ist in Figur 3 lediglich beispielhaft schematisch ein Verwendungsmuster P dargestellt, das eine bestimmte Verwendungsart V charakterisiert und typische Einstellungen bzw. zeitliche Verläufe eines oder mehrerer Musterparameter M umfasst - hier z.B.
- die Muster-Amplitude A1*-A4* der Ausgangsspannung UA,
- die Muster-Frequenz f1*-f4* der Ausgangsspannung UA,
- die Muster-Aktivierungsdauern DA1*- DA4*,
- die Muster-Deaktivierungsdauern DD1*-DD3*,
- die Muster-Gesamtdauer DE* der Behandlung.

Wie bereits erläutert, ist es auch möglich, die geplante, bevorstehende Verwendungsart V vorzugeben, so dass auf eine Ermittlung während der Anwendung verzichtet werden kann und bereits zum Startzeitpunkt ts die Verwendungsart V des Chirurgieinstruments 12 bekannt ist.

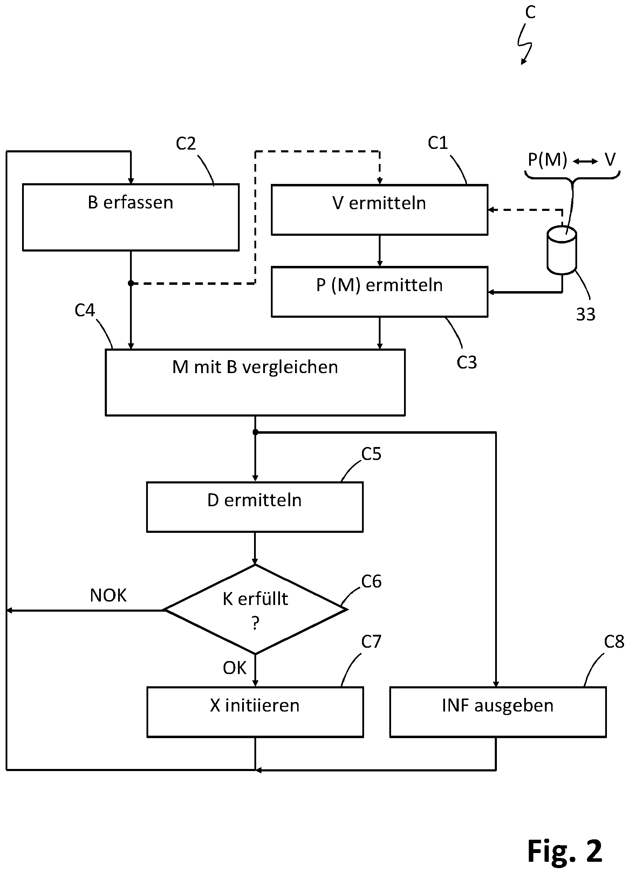

Unter Bezugnahme auf Figur 2 wird ein Beispiel eines Verfahrens C zum Betreiben bzw. Verwenden des Chirurgiesystems 10 erläutert.

In einem ersten Verfahrensschritt C1 wird die Verwendungsart V identifiziert, mit der das Chirurgiegerät 11 bei einem bevorstehenden geplanten chirurgischen Eingriff verwendet werden soll. Beispielsweise kann die geplante Verwendungsart V über die Bedienschnittstelle 34 oder das Datenmanagementsystem 30 vorgegeben werden.

Ab dem Startzeitpunkt ts werden der Betriebsparameter B bzw. wenigstens einer der Betriebsparameter B, mehrere Betriebsparameter B oder alle Betriebsparameter B erfasst, die das Chirurgiesystem 10 für den Betrieb des Chirurgieinstruments 12 einstellt. Der zweite Verfahrensschritt C2 kann - wie vorstehend erläutert - bei einer Abwandlung des in Figur 2 dargestellten Verfahrens (gestrichelt veranschaulicht) auch zeitlich vor dem ersten Verfahrensschritt C1 stattfinden, um die Verwendungsart V basierend auf einem Mustervergleich mit dem im Datenbankspeicher 33 verfügbaren Verwendungsmustern P auf Ähnlichkeit zu prüfen.

Nachdem die Verwendungsart V des Chirurgieinstruments 12 identifiziert wurde, kann zu dieser Verwendungsart V ein zugeordnetes Verwendungsmuster P durch Zugriff auf den Datenbankspeicher 33 ermittelt werden (dritter Verfahrensschritt C3) .

Das Verwendungsmuster P kann beispielsweise einen typischen oder idealen Verlauf eines chirurgischen Eingriffs zwischen dem Startzeitpunkt ts und dem Endzeitpunkt te mit dem jeweils zugehörigen Einstellungen des wenigstens einen Betriebsparameters B beschreiben. Die durch das Verwendungsmuster P definierten Musterparameter M stellen dabei Vergleichswerte oder Sollwerte für die Betriebsparameter B dar, die das Chirurgiegerät 11 während des chirurgischen Eingriffs dem Chirurgieinstrument 12 bereitstellt.

In einem vierten Verfahrensschritt C4 kann dann der im zweiten Verfahrensschritt C2 erfasste wenigstens eine Betriebsparameter B mit dem jeweils zugehörigen Musterparameter M des Verwendungsmusters P verglichen werden. Zwischen einem oder mehreren der verfügbaren Paare aus jeweils einem Betriebsparameter B und einem zugeordneten Musterparameter M kann dann eine Abweichung D ermittelt werden (fünfter Verfahrensschritt C5).

Im Anschluss an den fünften Verfahrensschritt C5 kann die ermittelte Abweichung D bzw. jede ermittelte Abweichung D daraufhin geprüft werden, ob ein zugeordnetes Abweichungskriterium K erfüllt ist (sechster Verfahrensschritt C6). Ist das Abweichungskriterium K nicht erfüllt (Verzweigung "NOK" aus dem sechsten Verfahrensschritt C6), kann das Verfahren C wieder mit der Erfassung des wenigstens einen Betriebsparameters B im zweiten Verfahrensschritt C2 fortgesetzt werden.

Ist jedoch das Abweichungskriterium K erfüllt (Verzweigung "OK" aus dem sechsten Verfahrensschritt C6), wird das Verfahren C in einem siebten Verfahrensschritt C7 fortgesetzt. Im siebten Verfahrensschritt C7 wird eine geeignete Maßnahme X durch die Auswerteeinheit 28 des Chirurgiegeräts 11 ausgelöst oder durchgeführt. Dabei sind beliebige verschiedene Maßnahmen X möglich, wie beispielsweise das Ausgeben einer Warnung oder Information über die Bedienschnittstelle 34 an eine Bedienperson und/oder das automatische Anpassen wenigstens eines Betriebsparameters B, um die betreffende Abweichung D zum jeweils zugeordneten Musterparameter M zu reduzieren.

Beispielsweise können eine oder mehrere der folgenden Maßnahmen X initiiert bzw. durchgeführt werden:
- Ausgeben einer Warnung oder Information über die Abweichung D;
- Ausgeben einer Empfehlung über eine Anpassung eines oder mehrerer der einstellbaren Betriebsparameter B;
- automatisches Ändern eines oder mehrerer der einstellbaren Betriebsparameter B.

Zusätzlich oder alternativ zu den Verfahrensschritten C5-C7 kann im Anschluss an den vierten Verfahrensschritt C4 in einem achten Verfahrensschritt C8 eine Maßnahme initiiert oder durchgeführt werden, bei der eine oder mehrere Informationen INF ausgegeben werden, beispielsweise über die Bedienschnittstelle 34. Die im achten Verfahrensschritt C8 definierte Maßnahme kann unabhängig von einer ermittelten Abweichung D sein. Sie kann optional auch unter Berücksichtigung der Abweichung D als Maßnahme im siebten Verfahrensschritt C7 ausgegeben werden. Die Information INF kann eine oder mehrere der folgenden Informationen umfassen:
- eine Information betreffend einen oder mehrere während des weiteren Verlaufs des chirurgischen Eingriffs bis zum Endzeitpunkt te bevorstehende Schritte des chirurgischen Eingriffs;
- eine Information betreffend eine oder mehrere benötigte Ressourcen für einen oder mehrere durchzuführende Schritte des chirurgischen Eingriffs;
- eine Information betreffend die Zeitdauer für einen, mehrere oder alle noch auszuführenden Schritte des chirurgischen Eingriffs;
- die Übermittlung des voraussichtlichen Endzeitpunkts te des chirurgischen Eingriffs an das Datenmanagementsystem 30;
- das Anfordern von einer oder mehreren Ressourcen über das Datenmanagementsystem 30.

In einer weiteren Ausgestaltung der Erfindung kann der gesamte Ablauf eines chirurgischen Eingriffs von einem Startzeitpunkt ts bis zu einem Endzeitpunkt te erfasst und gespeichert werden, beispielsweise in einem optional vorhandenen internen Speicher 40 des Chirurgiegeräts 11 und/oder im Datenmanagementsystem 30. Dies kann zu Dokumentationszwecken, zum Zwecke der Bewertung oder der Fortbildung bzw. des Trainings von Chirurgiepersonal erfolgen.

Das Chirurgiesystem 10 wurde vorstehend beispielhaft anhand eines Elektrochirurgiesystems mit einem Elektrochirurgieinstrument 13 erläutert. Das Chirurgiesystem 10 kann bei abgewandelten Ausführungsbeispielen auch ein Wasserstrahl-Chirurgieinstrument oder ein Ultraschall-Chirurgieinstrument oder ein anderes Chirurgieinstrument 12 sein. Es ist auch möglich, während eines chirurgischen Eingriffs mehrere unterschiedliche Chirurgieinstrumente 12 zu betreiben und zu verwenden.

Die Erfindung betrifft ein Chirurgiesystem 10 sowie ein Verfahren C zu dessen Betrieb. Das Chirurgiesystem 10 hat ein Chirurgieinstrument 12, beispielsweise ein Elektrochirurgieinstrument 13, das an ein Chirurgiegerät 11 angeschlossen und von diesem gesteuert wird. Das Chirurgiegerät 11 steuert wenigstens einen Betriebsparameter B des Chirurgieinstruments 12. Der Betriebsparameter B kann beispielsweise ein Parameter eines elektrischen Ausgangssignals sein, wie beispielsweise eine Amplitude und/oder eine Frequenz und/oder eine Kurvenform. Das Chirurgiesystem 10 hat eine Auswerteeinheit 28, die dazu eingerichtet ist, eine aktuell durchgeführte oder geplante Verwendungsart V des Chirurgieinstruments 12 bzw. des gesamten Chirurgiesystems 10 zu identifizieren, wobei die Verwendungsart V charakteristisch ist für die Art der durchzuführenden Operation. Der identifizierten Verwendungsart V ist wenigstens ein Musterparameter M zugeordnet, der mit jeweils einem zugeordneten Betriebsparameter B verglichen werden kann. Basierend auf dem Resultat des Vergleichs kann wenigstens eine Maßnahme X eingeleitet werden, beispielsweise das Ausgeben einer Information INF und/oder das Anpassen wenigstens eines Betriebsparameters B.

### Bezugszeichenliste:

- 10: Chirurgiesystem
- 11: Chirurgiegerät
- 12: Chirurgieinstrument
- 13: Elektrochirurgieinstrument

- 14: Elektrode
- 15: Gewebe
- 16: Instrumentenleitung
- 17: Neutralelektrode
- 18: Neutralelektrodenleitung
- 19: Versorgungseinheit
- 20: Generator
- 21: Fluidquelle
- 22: Sensor
- 23: Steuereinheit

- 28: Auswerteeinheit
- 29: Kommunikationsnetzwerk
- 30: Datenmanagementsystem
- 31: Recheneinheit
- 32: Datenbank
- 33: Datenbankspeicher
- 34: Bedienschnittstelle

- 40: interner Speicher

- A1: erste Amplitude
- A1*: erste Muster-Amplitude
- A2: zweite Amplitude
- A2*: zweite Muster-Amplitude
- A3: dritte Amplitude
- A3*: dritte Muster-Amplitude
- A4: vierte Amplitude
- A4*: vierte Muster-Amplitude
- B: Betriebsparameter
- C: Verfahren
- C1: erster Verfahrensschritt
- C2: zweiter Verfahrensschritt
- C3: dritter Verfahrensschritt
- C4: vierter Verfahrensschritt
- C5: fünfter Verfahrensschritt
- C6: sechster Verfahrensschritt
- C7: siebter Verfahrensschritt
- C8: achter Verfahrensschritt
- D: Abweichung
- DA: Aktivierungsdauer
- DA1: erste Aktivierungsdauer
- DA1*: erste Muster-Aktivierungsdauer
- DA2: zweite Aktivierungsdauer
- DA2*: zweite Muster-Aktivierungsdauer
- DA3: dritte Aktivierungsdauer
- DA3*: dritte Muster-Aktivierungsdauer
- DA4: vierte Aktivierungsdauer
- DA4*: vierte Muster-Aktivierungsdauer
- DD: Deaktivierungsdauer
- DD1: erste Deaktivierungsdauer
- DD1*: erste Muster-Deaktivierungsdauer
- DD2: zweite Deaktivierungsdauer
- DD2*: zweite Muster-Deaktivierungsdauer
- DD3: dritte Deaktivierungsdauer
- DD3*: dritte Muster-Deaktivierungsdauer
- DE: Gesamtdauer der Verwendung des Chirurgieinstruments
- DE*: Muster-Gesamtdauer der Verwendung des Chirurgieinstruments
- F: Fluid
- f1: erste Frequenz
- f1*: erste Muster-Frequenz
- f2: zweite Frequenz
- f2*: zweite Muster-Frequenz
- f3: dritte Frequenz
- f3*: dritte Muster-Frequenz
- f4: vierte Frequenz
- f4*: vierte Muster-Frequenz
- IA: elektrischer Ausgangsstrom
- INF: Information
- K: Abweichungskriterium
- M: Musterparameter
- P: Verwendungsmuster
- DA1*: erste Muster-Aktivierungsdauer
- DA2*: zweite Muster-Aktivierungsdauer
- DA3*: dritte Muster-Aktivierungsdauer
- DA4*: vierte Muster-Aktivierungsdauer
- DD1*: erste Muster-Deaktivierungsdauer
- DD2*: zweite Muster-Deaktivierungsdauer
- DD3*: dritte Muster-Deaktivierungsdauer
- S: Steuersignal
- ts: Startzeitpunkt
- te: Endzeitpunkt
- UA: elektrische Ausgangsspannung
- V: Verwendungsart
- X: Maßnahme

## Patentansprüche

1. Chirurgiesystem (10) aufweisend:
- ein Chirurgieinstrument (12), insbesondere ein Elektrochirurgieinstrument (13),
- ein Chirurgiegerät (11), das zum Anschließen des Chirurgieinstruments (12) eingerichtet ist und das eine Steuereinheit (23) aufweist, die zur Steuerung wenigstens eines Betriebsparameters (B) des Chirurgieinstruments (12) eingerichtet ist,
- eine mit dem Chirurgiegerät (11) kommunikationsverbundene Auswerteeinheit (28), die eingerichtet ist zur Identifikation einer aktuell durchgeführten oder einer geplanten Verwendungsart (V) des Chirurgieinstruments (12), zur Zuordnung wenigstens eines Musterparameters (M) zur Verwendungsart (V), zum Vergleich des wenigstens einen Betriebsparameters (B) der Verwendungsart (V) mit dem wenigstens einen Musterparameter (M) und zum Ermitteln und Initiieren wenigstens einer durchzuführenden Maßnahme (X, INF) abhängig von einem Resultat des Vergleichs.

2. Chirurgiesystem nach Anspruch 1, wobei die Verwendungsart (V) charakterisiert ist durch eine zeitliche Reihenfolge von Einstellungen des wenigstens einen Betriebsparameters (B).

3. Chirurgiesystem nach Anspruch 1 oder 2, wobei der wenigstens eine Betriebsparameter (B) ein dem Chirurgieinstrument bereitgestelltes elektrisches Signal und/oder ein Fluidparameter eines dem Chirurgieinstrument bereitgestellten Fluids und/oder ein dem Chirurgieinstrument bereitgestelltes optisches Signal aufweist.

4. Chirurgiesystem nach einem der vorhergehenden Ansprüche, wobei der wenigstens eine Betriebsparameter (B) einen oder mehrere der nachfolgenden Betriebsparameter (B) aufweisen kann:
- einen Amplitude (A1, A2, A3, A4) und/oder eine Frequenz (f1, f2, f3, f4) einer dem Chirurgieinstrument (12) bereitgestellten elektrischen Ausgangssignals,
- ein Crest-Faktor des elektrischen Ausgangssignals,
- einen Tastgrad des elektrischen Ausgangssignals, wenn das elektrische Ausgangssignal pulsweitenmoduliert ist,
- eine Kurvenform und/oder ein Mode des elektrischen Ausgangssignals,
- einen Fluiddruck und/oder ein Volumenstrom eines dem Chirurgieinstrument (12) bereitgestellten Fluids (F) .

5. Chirurgiesystem nach einem der vorhergehenden Ansprüche, wobei die Auswerteeinheit (28) dazu eingerichtet ist, als die Maßnahme oder als wenigstens eine der Maßnahmen den Betriebsparameter (B) oder wenigstens einen der Betriebsparameter (B) zu verändern.

6. Chirurgiesystem nach einem der vorhergehenden Ansprüche, wobei die Auswerteeinheit (28) außerdem dazu eingerichtet ist, als das Resultat oder als eines der Resultate des Vergleichs eine Abweichung (D) zwischen dem Betriebsparameter (B) oder wenigstens einem der Betriebsparameter (B) und dem jeweils zugeordneten Musterparameter (M) zu ermitteln.

7. Chirurgiesystem nach Anspruch 6, wobei die Auswerteeinheit (28) außerdem dazu eingerichtet ist, dann die Maßnahme oder eine der Maßnahmen zu ermitteln und zu initiieren, wenn die Abweichung (D) ein Abweichungskriterium (K) erfüllt.

8. Chirurgiesystem nach einem der vorhergehenden Ansprüche, wobei die Auswerteeinheit (28) dazu eingerichtet ist, als die Maßnahme oder als wenigstens eine der Maßnahmen eine Information (INF) auszugeben, wobei die Information (INF) eine oder mehrere der folgenden Informationen enthalten kann:
- Warnung über eine Abweichung des wenigstens einen Betriebsparameters (B) von dem wenigstens einen Musterparameter (M),
- Empfehlung betreffend eine Änderung des Betriebsparameters (B) oder wenigstens eines der Betriebsparameter (B),
- Information betreffend einen oder mehrere nachfolgend auszuführende Schritte,
- Information betreffend eine oder mehrere benötigte Ressourcen für einen oder mehrere nachfolgend auszuführende Schritte,
- Information betreffend eine Zeitdauer für einen oder mehrere nachfolgend auszuführende Schritte.

9. Chirurgiesystem nach einem der vorhergehenden Ansprüche, wobei das Chirurgiegerät (11) mittels eines Kommunikationsnetzwerkes (29) mit einem Datenmanagementsystem (30) verbunden ist.

10. Chirurgiesystem nach Anspruch 9, wobei die Auswerteeinheit (28) dazu eingerichtet ist, von dem Datenmanagementsystem (30) vor Beginn der Verwendung des Chirurgieinstruments (12) eine Information zu erhalten, die die geplanten Verwendungsart (V) identifiziert.

11. Chirurgiesystem nach Anspruch 9 oder 10, wobei das Chirurgiegerät (11) dazu eingerichtet ist, den Betriebsparameter (B) oder wenigstens einen der Betriebsparameter (B), der bei einer Verwendung des Chirurgieinstruments (12) auftritt, zu speichern und/oder an das Datenmanagementsystem (30) zu übertragen.

12. Chirurgiesystem nach einem der vorhergehenden Ansprüche, wobei die Auswerteeinheit (28) dazu eingerichtet ist, den wenigstens einen Betriebsparameter (B) bei einer Verwendung des Chirurgieinstruments (12) zu ermitteln und mit dem wenigstens einen Musterparameter (M) von mehreren Verwendungsmustern (P) zu vergleichen und bei Feststellung einer ausreichenden Ähnlichkeit zwischen dem wenigstens einen Betriebsparameter (B) und dem wenigstens einen Musterparameter (M) die aktuell durchgeführte Verwendungsart (V) zu identifizieren.

13. Chirurgiesystem nach einem der vorhergehenden Ansprüche, wobei die Auswerteeinheit (28) dazu eingerichtet ist, als Resultat des Vergleichs eine Bewertung der durchgeführten Verwendung des Chirurgieinstruments (12) zu ermitteln.

14. Verfahren zum Betreiben eines Chirurgiesystems (10), wobei das Chirurgiesystem (10) ein Chirurgiegerät (11) und ein daran angeschlossenes Chirurgieinstrument (12) aufweist, wobei das Chirurgiegerät (11) eine Steuereinheit (23) aufweist, die zur Steuerung wenigstens eines Betriebsparameters (B) des Chirurgieinstruments (12) eingerichtet ist, wobei das Verfahren umfasst:
- Identifikation der aktuell durchgeführten oder geplanten Verwendungsart (V) des Chirurgieinstruments (12),
- Zuordnung wenigstens eines Musterparameters (M) zur Verwendungsart (V),
- Vergleich des wenigstens einen Betriebsparameters (B) der Verwendungsart (V) mit dem wenigstens einen Musterparameter (M) und
- Ermitteln und Initiieren wenigstens einer durchzuführenden Maßnahme (X, INF) abhängig von einem Resultat des Vergleichs.
